(19) Europäisches Patentamt / European Patent Office / Office européen des brevets

(11) **EP 4 715 381 A1**

(12) **EUROPEAN PATENT APPLICATION**
published in accordance with Art. 153(4) EPC

(43) Date of publication:
**25.03.2026 Bulletin 2026/13**

(21) Application number: **24807211.8**

(22) Date of filing: **14.05.2024**

(51) International Patent Classification (IPC):
***G01N 33/50*** (2006.01)

(52) Cooperative Patent Classification (CPC):
**G01N 33/50**

(86) International application number:
**PCT/JP2024/017846**

(87) International publication number:
**WO 2024/237259 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.05.2023 JP 2023081585**

(71) Applicants:
• **SHIMADZU CORPORATION**
**Kyoto-shi, Kyoto 604-8511 (JP)**
• **ST. LUKE'S INTERNATIONAL UNIVERSITY**
**Tokyo 104-0044 (JP)**

(72) Inventors:
• **AOKI, Yutaka**
**Kyoto-shi, Kyoto 604-8511 (JP)**

• **WATANABE, Makoto**
**Kyoto-shi, Kyoto 604-8511 (JP)**
• **SATO, Taka-Aki**
**Kyoto-shi, Kyoto 604-8511 (JP)**
• **KIMURA, Takeshi**
**Tokyo 104-0044 (JP)**
• **KOSHIZAKA, Takuya**
**Tokyo 104-0044 (JP)**
• **OHTAKE, Jyunya**
**Tokyo 104-0044 (JP)**
• **KUMAKURA, Yasuhisa**
**Tokyo 104-0044 (JP)**

(74) Representative: **Müller-Boré & Partner**
**Patentanwälte PartG mbB**
**Friedenheimer Brücke 21**
**80639 München (DE)**

(54) **METHOD FOR EVALUATING ONSET RISK OF NON-ALCOHOLIC FATTY LIVER DISEASE, AND BIOMARKERS**

(57) To provide a highly reliable method for evaluating an onset risk of NAFLD and a biomarker for use in the method. A method for evaluating an onset risk of non-alcoholic fatty liver disease includes: an acquisition step of acquiring a sample derived from a body fluid of a subject; a detection step of detecting 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan in the sample; and a use step of using all detection amounts of the 14 kinds of compounds obtained in the detection step.

FIG. 1

TRAINING SET (AREA UNDER CURVE = 0.80)   TEST SET (AREA UNDER CURVE = 0.76)

**Description**

TECHNICAL FIELD

The present invention relates to a method for evaluating an onset risk of nonalcoholic fatty liver disease (NAFLD), and a biomarker for use in the method.

BACKGROUND ART

NAFLD refers to fatty liver conditions not caused by alcohol, viral infection, or similar factors. NAFLD encompasses simple steatosis, in which fat just accumulates in hepatocytes, and nonalcoholic steatohepatitis (NASH), in which hepatocytes become damaged and lose function, leading to inflammation and fibrosis from fatty liver with a risk of progressing to cirrhosis or liver cancer. Since simple steatosis can progress to nonalcoholic steatohepatitis, the diagnosis of NAFLD, including NASH and simple steatosis, is important. Then, if it is possible to diagnose whether or not a medical examinee is at present suffering from NAFLD, and if it is possible to diagnose whether or not the onset risk, that is, whether or not the possibility of onset of NAFLD in the future is high, it is meaningful since it is possible to recommend preventive measures against NAFLD to the medical examinee.

As a method for predicting such an onset risk, for example, a prediction method of measuring the total amount of acylcarnitine in a sample (Patent Document 1), a prediction or diagnostic method of measuring the concentration of growth differentiation factor 15 protein in a sample (Patent Document 2), a method of comparing the levels of trimethylamine-containing compounds (Patent Document 3), and the like have been proposed. However, the methods described above were validated on relatively small cohorts ( approximately 200 individuals), which limits their reliability.

On the other hand, as a method for analyzing several thousand people, a method for predicting the NAFLD risk has been proposed in which a single nucleotide polymorphism at position 27730940 of the GCKR gene located on human chromosome 2, a single nucleotide polymorphism in a linkage disequilibrium state with the single nucleotide polymorphism, or the like is detected (Patent Document 4).

PRIOR ART DOCUMENTS

PATENT DOCUMENTS

Patent Document 1: JP 2020-34561 A
Patent Document 2: JP 2020-528138 W
Patent Document 3: JP 2012-528340 W
Patent Document 4: JP 2020-120625 A

SUMMARY OF THE INVENTION

PROBLEMS TO BE SOLVED BY THE INVENTION

However, in the method described in Patent Document 4, since a genetic analysis method using primers and probes that hybridize to specific base sequences (SNP) is adopted, the prediction of onset of a disease is based on a genetic background (diathesis) as a base, and internal and external environmental factors are not considered. Therefore, alternative methods to the genetic analysis method are desired.

Therefore, it is an object of the present invention to provide a highly reliable method for evaluating an onset risk of NAFLD and a biomarker used in the method.

MEANS FOR SOLVING THE PROBLEMS

A method for evaluating an onset risk of NAFLD according to a first aspect of the present invention includes: an acquisition step of acquiring a sample derived from a body fluid of a subject; a detection step of detecting 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan in the sample; and a use step of using all detection amounts of the 14 kinds of compounds obtained in the detection step.

EFFECTS OF THE INVENTION

The method of evaluating onset risk and the biomarkers of the first aspect of the present invention enable a highly reliable prediction of future NAFLD onset.

BRIEF DESCRIPTION OF THE DRAWINGS

FIG. 1 is a graph evaluating the performance of a predictive discriminant in Example 1 (31 kinds of specific compounds) using an ROC curve, and the right figure is a graph in which a sample cohort is a training set while the right figure is a graph in which a sample cohort is a test set. In FIG. 1, the vertical axis represents sensitivity, and the horizontal axis represents non-specificity (1-specificity).
FIG. 2 is a graph evaluating the performance of the predictive discriminant in Examples using an ROC curve for a cohort different from FIG. 1.
FIG. 3 is a graph evaluating the performance of a predictive discriminant in Example 2 (21 kinds of specific compounds) using an ROC curve, and the right figure is a graph in which a sample cohort is a training set while the right figure is a graph in which a sample cohort is a test set.
FIG. 4 is a graph evaluating the performance of a predictive discriminant in Example 3 (15 kinds of specific compounds) using an ROC curve, and the right figure is a graph in which a sample cohort is a training set while the right figure is a graph in which a sample cohort is a test set.
FIG. 5 is a graph evaluating the performance of a predictive discriminant in Example 4 (14 kinds of specific compounds) using an ROC curve, and the right figure is a graph in which a sample cohort is a training set while the right figure is a graph in which a sample cohort is a test set.

MODE FOR CARRYING OUT THE INVENTION

1. First Embodiment

A method for evaluating an onset risk of NAFLD according to a first embodiment of the present invention is a method for evaluating the presence or absence of the onset of NAFLD in the future, in which 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan are used as biomarkers for evaluating the onset risk of NAFLD. Specifically, the evaluation method of the first embodiment includes an acquisition step, a detection step, and a use step. Hereinafter, each step will be described in detail.

(Acquisition Step)

In this step, a sample derived from a body fluid of a subject is obtained.
Examples of the body fluid include blood, urine, saliva, sweat, sputum, and feces. The blood includes whole blood, plasma, serum, and the like. The body fluid is preferably blood, more preferably serum. Blood sampling is minimally invasive, and blood is readily available as a sample to be subjected to screening in medical examinations and the like.
The sample is temporarily stored before the detection step. The storage method may be any of frozen storage, refrigerated storage, and room-temperature storage. When serum or plasma is used as the sample, it may be stored in the form of whole blood, or in the form of serum or plasma obtained by centrifuging whole blood.

(Detection Step)

In this step, at least all of 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan are detected from the sample. Hereinafter, these 14 kinds of compounds are also collectively referred to as specific compounds.
In this embodiment, the phrase "detecting a compound" refers to directly or indirectly detecting a specific compound contained in a sample for quantification, and specifically includes not only detecting the specific compound itself but also performing treatment such as ionization, dissociation, and derivatization on the specific compound to detect the treated specific compound (e.g., ionized matter, dissociated matter, or derivative).
The detection method may be any method capable of quantifying a specific compound contained in the sample, and examples thereof include gas chromatography (GC), liquid chromatography (LC), mass spectrometry (MS), gas chromatography/mass spectrometry (GC/MS), liquid chromatography/mass spectrometry (LC/MS), nuclear magnetic resonance spectroscopy, and immunological detection. From the viewpoint of enabling detection with high sensitivity for a blood sample, the detection method preferably involves the use of mass spectrometry (MS, LC/MS, or GC/MS), more preferably GC/MS.

When mass spectrometry is employed, examples of ionization means include electron ionization (EI), electrospray ionization (ESI), and atmospheric pressure chemical ionization (APCI). Examples of mass separation means include a quadrupole type, a magnetic sector type, a time-of-flight type, an ion trap type, and an ion cyclotron resonance type. Further examples thereof include a tandem mass separation type such as a triple quadrupole type (Q-Q), a tandem time-of-flight type (TOF-TOF), a quadrupole-time-of-flight type (Q-TOF), a quadrupole-ion trap type (Q-IT), a quadrupole-ion cyclotron resonance type (Q-ICR), and an ion trap-time-of-flight type (IT-TOF).

The sample may be subjected to pretreatment before the detection step is performed. Examples of the pretreatment include treatments for purification, derivatization, and the like. For example, when detection is performed by mass spectrometry using serum or the like, deproteinization treatment, derivatization, or the like is preferably performed.

The deproteinization treatment is performed, for example, by mixing each of an extraction solvent such as a chloroform solvent, water, and the like with a sample, centrifuging the mixture, and removing a precipitate of the mixture after centrifugation.

Examples of the derivatization include silylation, such as trimethylsilylation, dimethylsilylation, and halomethylsilylation; esterification such as methyl esterification; acylation; and alkylation. From the viewpoints of derivatizing a wide range of target compounds, reacting with many compounds containing active hydrogen, and producing a peak that facilitates mass spectrometry analysis, the derivatization is preferably silylation, particularly preferably trimethylsilylation. That is, it is preferable to use the detection amount of a derivative of the specific compound, particularly, the trimethylsilylated derivative of the specific compound.

When trimethylsilylation is employed, oximation may be performed, for example, by adding a methoxyamine solution to a freeze-dried sample, followed by the addition of a trimethylsilylating agent. Examples of the trimethylsilylating agent include N,O-bis(trimethylsilyl)trifluoroacetamide (BSTFA) and N-methyl-N-trimethylsilyltrifluoroacetamide (MSTFA).

Examples of the trimethylsilylated derivative of the specific compound include:

bis(trimethylsilyl)lactic acid (Lactic acid-2TMS),
bis(trimethylsilyl)alanine (Alanine-2TMS),
trimethylsilylacetylglycine (Acetylglycine-TMS),
bis(trimethylsilyl)isoleucine (Isoleucine-2TMS),
bis(trimethylsilyl)proline (Proline-2TMS),
tris(trimethylsilyl)serine (Serine-3TMS),
bis(trimethylsilyl)glutaric acid (Glutaric acid-2TMS),
bis(trimethylsilyl)$\alpha$-ketoglutaric acid-methyloxime (a-Ketoglutaric acid-methyloxime-2TMS),
tris(trimethylsilyl)creatinine (Creatinine-3TMS),
tris(trimethylsilyl)glutamic acid (Glutamic acid-3TMS),
tris(trimethylsilyl)asparagine (Asparagine-3TMS),
pentakis(trimethylsilyl)fructose-methyloxime (Fructose-methyloxime-5TMS),
tetrakis(trimethylsilyl) uric acid (Uric acid-4TMS), and
bis(trimethylsilyl)tryptophan (Tryptophan-2TMS). When a specific compound is derivatized, a plurality of derivatives (e.g., Glycine-2TMS and Glycine-3TMS) may be obtained for one specific compound (e.g., Glycine described later). In such a case, only one of the plurality of derivatives may be arbitrarily selected, or a plurality of derivatives may be selected. When a plurality of derivatives is selected, a predetermined coefficient (described later) is set for each of the plurality of derivatives in a use step described later. In addition, when the coefficient is set, the detection amounts of a plurality of derivatizations may be added up. Then, the detection amounts of the plurality of derivatives and a plurality of predetermined coefficients corresponding thereto are substituted into the predictive discriminant. Even when a plurality of derivatives is used from one specific compound, the number of specific compounds selected is treated as one.

During the pretreatment, it is preferable to mix stable isotopes of specific compounds and/or internal standard substances with the sample. The absolute amount of a specific compound contained in a body fluid, such as blood, can be measured by mixing stable isotopes. Further, the accuracy of quantification by mass spectrometry can be enhanced by mixing the internal standard substance. Examples of the stable isotopes include compounds in which one or more carbon atoms, hydrogen atoms, nitrogen atoms, or oxygen atoms in the molecule are substituted with a carbon stable isotope (12C or 13C), a hydrogen stable isotope (1H, 2H, or D), a nitrogen stable isotope (14N or 15N), or an oxygen stable isotope (16O, 17O, or 18O). The internal standard substance may be any substance that is not originally contained in the body fluid, and examples thereof include 2-isopropylmalic acid, ribitol, and tropic acid.

The pretreated sample is detected using the detection method. The conditions of the detection method may be appropriately set with reference to conditions recommended for the apparatus for use in the detection method (e.g., a GC/MS apparatus or an LC/MS apparatus).

As a result, the detection amount of each of the specific compounds contained in the sample is obtained. The detection amount is appropriately determined according to the detection method and detection apparatus, and may be output, for example, as a concentration; as a peak area or peak intensity in a mass spectrum or mass chromatogram; or as a light intensity. In such a case, the detection amount may be converted. For example, when the detection amount of the specific compound is output as the peak area of the mass spectrum, the peak area may be converted into the concentration. That is, the "detection amount" in the first embodiment includes not only the detection amount directly output from the detection apparatus but also the amount obtained by converting the detection amount into another parameter. Examples of the conversion include known methods such as an internal standard method and an absolute calibration curve method, and the conversion may be performed using any means such as an analysis function attached to a detection apparatus, another analysis apparatus, or a manual method. In the first embodiment, it is preferable to employ the concentration as the detection amount, and it is particularly preferable to employ the concentration ($\mu$g) of the specific compound present in 1 mL of serum.

(Use Step)

In this step, at least all detection amounts of the 14 kinds of compounds obtained in the detection step are used. Specifically, all of the detection amounts of 14 kinds of compounds in the specific compounds are used to evaluate, based on the use results, the risk of the subject suffering from NAFLD in the future. This use evaluates the high or low onset risk of NAFLD for the subject.

For example, detection amounts of all 14 kinds of specific compounds detected from a sample of a subject are used in a predetermined formula, specifically, substituted into the predictive discriminant. Subsequently, the result obtained from this analysis is utilized to assess the likelihood of the subject developing NAFLD in the future.

The predictive discriminant includes a sum (specifically, the second term on the right side of formula (1) to be described below) obtained by adding a value obtained by multiplying each of the detection amounts of the 14 kinds of specific compounds by a predetermined coefficient for each specific compound. That is, each detection amount of the compound (14 kinds) is multiplied by a specific coefficient determined in advance for each compound. As a result, 14 values are obtained for each compound, and therefore, the values are summed to derive a sum, and the sum is substituted into the predictive discriminant.

Examples of such a predictive discriminant include formulae (1) and (2) below.

[Equation 1]

$$[\text{Prediction Score}] = [\text{Constant K}] + \sum([\text{Coefficient of each compound}] \times [\text{Detection amount of each compound}]) \cdots (1)$$

$$[\text{Hazard ratio}] = e^{[\text{Prediction Score}]} \cdots (2)$$

Then, the calculation result by the predictive discriminant, that is, the hazard ratio is used as a criterion for determining whether or not the subject will suffer from NAFLD in the future (e.g., within 1 to 3 years). For example, when the hazard ratio is 4 or more, it is determined that the risk of morbidity is high, when the hazard ratio is 1 or more and less than 4, it is determined that the risk of morbidity is moderate, and when the hazard ratio is less than 1, it is determined that the possibility of morbidity is low.

Note that the constant K and the predetermined coefficient (14 kinds of coefficients) of each compound are obtained, for example, by a method of acquiring data obtained by measuring a large number of samples of healthy individuals and samples of NAFLD patients in advance, and calculating and verifying the data using a statistical machine learning method (LASSO or the like). In the first embodiment, as a result of the inventors already collecting and calculating a large amount of follow-up data of NAFLD morbidity within 3 years of healthy individuals, it is described in Table 2~8 of Examples described later, and constants K thereof and coefficients of each compound may be used.

In addition, as a method other than using the hazard ratio calculated by the above formulas (1) and (2), for example, it may be determined that the onset risk of NAFLD in the future is high or low on the basis of (i) a value using the detection amount of the specific compound detected from the sample of the subject (value of the subject) and a value using the detection amount of the specific compound detected from a sample of a person who remains healthy for a predetermined period after sampling or a person who is healthy at the time of sampling but suffers from NAFLD within a predetermined period (Value of normal group or value of NAFLD preliminary group), and (ii) whether the value of the subject is higher or lower than the value of the normal group or the value of the NAFLD preliminary group. Specifically, (i) a value obtained by substituting the detection amount of the subject into the second term on the right side of the above formula (1) is compared with a result obtained by substituting the detection amount of the normal group or the NAFLD preliminary group into the second term on the right side of the above formula (1). Then, (ii) when the value of the subject is equal to or higher (or lower) than the value of the NAFLD preliminary group (threshold of NAFLD onset), the subject may determine that the onset risk of NAFLD in the future is high, and when the value of the subject is equal to or lower (or higher) than the value of the normal group (normal threshold), the subject may determine that the onset risk of NAFLD in the future is low. A part or all of the present use step may be processed by a computer.

The evaluation method of the first embodiment can predict whether or not the subject suffers from NAFLD in the future. As a result, the level of the onset risk of NAFLD is evaluated for the subject. Therefore, for example, when a medical practitioner presents a determination result by performing this determination method to a doctor, the doctor can use the determination result for a policy of NAFLD prevention use for a medical examinee. Specifically, the treatment step may be performed after the evaluation method, particularly after the use step. The treatment step includes a known method in the treatment of NAFLD, and examples thereof include dietary therapy, exercise therapy, drug therapy, and surgical therapy. Diet therapy, exercise therapy, surgical therapy, and the like, which are aimed at weight reduction, are effective, and as the medication used in drug therapy, for example, antioxidants, diabetes therapeutic agents, and the like are effective. These therapies are applied in appropriate combination according to the situation of the subject.

2. Other Embodiments

In the first embodiment, the detection results of 14 kinds of specific compounds are used, but the present invention may include the detection amounts of 31 kinds of specific compounds, and for example, the detection amounts of other compounds may also be added. That is, detection amounts of 15 or more kinds of compounds may be used. Examples will be given below.

2-1. 31 Kinds of Specific Compounds

In the present embodiment, as a biomarker for evaluating the onset risk of NAFLD, pyruvic acid, 2-aminobutyric acid, succinic acid, threonine, decanoic acid, methionine, phenylalanine, xylose, arabinose, $\alpha$-aminoadipic acid, lysine, 1-hexadecanol, tyrosine, myoinositol, uridine, glycine, and leucine are used in addition to 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan. That is, 31 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, pyruvic acid, 2-aminobutyric acid, succinic acid, threonine, decanoic acid, methionine, phenylalanine, xylose, arabinose, $\alpha$-aminoadipic acid, lysine, 1-hexadecanol, tyrosine, myo-inositol, uridine, glycine, and leucine are used as the biomarkers for evaluating the onset risk of NAFLD. These 31 kinds of compounds are also collectively referred to as specific compounds.

This embodiment is similar to the above embodiment except that these 31 kinds of compounds are used as biomarkers. That is, the evaluation method of the present implementation step includes the acquisition step, the detection step, and the use step as described above in the first embodiment. The present embodiment also exhibits the same advantageous effects as the first embodiment.

In the detection step, examples of the trimethylsilylated derivatives of the 31 kinds of specific compounds include, in addition to trimethylsilylated derivatives of the 14 specific compounds,

trimethylsilyl pyruvic acid-methyloxime (pyruvic acid-methyloxime-TMS),
bis (trimethylsilyl) 2-aminobutyric acid-2TMS (2-Aminobutyric acid-2TMS)),
bis (trimethylsilyl) succinic acid-2TMS (Succinic acid-2TMS),
tris (trimethylsilyl) threonine (Threonine-3TMS),
trimethylsilyl decanoic acid (Decanoic acid-TMS),
bis (trimethylsilyl) methionine (Methionine-2TMS),
bis (trimethylsilyl) phenylalanine (Phenylalanine-2TMS),
tetrakis (trimethylsilyl) xylose-methyloxime (Xylose-methyloxime -4TMS),
tetrakis (trimethylsilyl) arabinose-methyloxime(Arabinose-methyloxime-4TMS),
tris (trimethylsilyl) $\alpha$-aminoadipic acid (Aminoadipic acid-3TMS),
tetrakis (trimethylsilyl) lysine (Lysine-4TMS),
trimethylsilyl 1-hexadecanol (1-Hexadecanol-TMS),

tris (trimethylsilyl) tyrosine (Tyrosine-3TMS),
hexakis (trimethylsilyl) myo-inositol (myo-inositol -6TMS),
tetrakis (trimethylsilyl) uridine (Uridine-4TMS),
bis (trimethylsilyl) glycine (Glycine-2TMS) and/or tris (trimethylsilyl) glycine (Glycine-3TMS), and
bis (trimethylsilyl) leucine (Leucine -2TMS). In the use step, the detection amounts of at least the 31 kinds of compounds (or derivatives thereof)) are used. Specifically, values obtained by multiplying each detection amount (concentration or the like) of at least 31 kinds of specific compounds (or derivatives thereof) by a predetermined coefficient for each specific compound are added together, and the sum obtained thereby may be used for the predictive discriminant.

2-2. 21 Kinds of Specific Compounds

In the present embodiment, as a biomarker for evaluating the onset risk of NAFLD, 3-hydroxybutyric acid, leucine, glycine, methionine, glucose, tyrosine, and myo-inositol are further used in addition to 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan. That is, 21 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, 3-hydroxybutyric acid, leucine, glycine, methionine, glucose, tyrosine, and myo-inositol are used as the biomarker for evaluating the onset risk of NAFLD. These 21 kinds of compounds are also collectively referred to as specific compounds.

This embodiment is similar to the above embodiment except that these 21 kinds of compounds are used as biomarkers. That is, the evaluation method of the present implementation step includes the acquisition step, the detection step, and the use step as described above in the first embodiment. The present embodiment also exhibits the same advantageous effects as the first embodiment.

In the detection step, examples of the trimethylsilylated derivatives of the 21 kinds of specific compounds include, in addition to trimethylsilylated derivatives of the 14 specific compounds,

bis (trimethylsilyl) 3-hydroxybutyric acid (3-Hydroxybutyric acid-2TMS),
bis (trimethylsilyl) leucine (Leucine-2TMS),
bis (trimethylsilyl) glycine (Glycine-2TMS) and/or tris (trimethylsilyl) glycine (Glycine-3TMS),
bis (trimethylsilyl) methionine (Methionine-2TMS),
pentakis (trimethylsilyl) glucose-methyloxime (Glucose-methyloxime-5TMS),
tris (trimethylsilyl) tyrosine (Tyrosine-3TMS), and
hexakis (trimethylsilyl) myo-inositol (myo-inositol-6TMS). In the use step, the detection amounts of at least the 21 kinds of compounds are used. Specifically, values obtained by multiplying each detection amount of at least 21 kinds of specific compounds by a predetermined coefficient for each specific compound are added together, and the sum obtained thereby may be used for the predictive discriminant.

2-3. 15 Kinds of Specific Compounds

In the present embodiment, as a biomarker for evaluating the onset risk of NAFLD, glycine is further used in addition to 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan. That is, 15 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and glycine are used as biomarkers for evaluating the onset risk of NAFLD. These 15 kinds of compounds are also collectively referred to as specific compounds.

This embodiment is similar to the above embodiment except that these 15 kinds of compounds are used as biomarkers. That is, the evaluation method of the present implementation step includes the acquisition step, the detection step, and the use step as described above in the first embodiment. The present embodiment also exhibits the same advantageous effects as the first embodiment.

In the detection step, examples of the trimethylsilylated derivatives of the 15 kinds of specific compounds include bis (trimethylsilyl) glycine (Glycine-2TMS) and/or tris (trimethylsilyl) glycine (Glycine-3TMS) in addition to the trimethylsilylated derivatives of the 14 kinds of specific compounds. In the use step, the detection amounts of at least the 15 kinds of compounds are used. Specifically, values obtained by multiplying each detection amount of at least 15 kinds of specific compounds by a predetermined coefficient for each specific compound are added together, and the sum obtained thereby may be used for the predictive discriminant.

3. Aspects

It is understood by those skilled in the art that the above-described illustrative embodiments are specific examples of the following aspects.

(Clause 1) A method for evaluating an onset risk of NAFLD according to an aspect may include: a detection step of detecting 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid and tryptophan in the sample; and a use step of using all detection amounts of the 14 kinds of compounds obtained in the detection step. This evaluation method enables prediction of whether or not a subject will suffer from NAFLD in the future.

(Clause 2) In the evaluation method according to clause 1, glycine may be detected in addition to the 14 kinds of compounds, and all of the 15 kinds of detection amounts obtained by the detection step may be used in the use step. This evaluation method enables more accurate prediction of whether or not a subject suffers from NAFLD.

(Clause 3) In the evaluation method according to clause 1, 3-hydroxybutyric acid, leucine, glycine, methionine, glucose, tyrosine, and myo-inositol may be detected in addition to the 14 kinds of compounds, and all of the 21 kinds of detection amounts obtained by the detection step may be used in the use step. This evaluation method enables more accurate prediction of whether or not a subject suffers from NAFLD in the future.

(Clause 4) In the evaluation method according to clause 1, pyruvic acid, 2-aminobutyric acid, succinic acid, threonine, decanoic acid, methionine, phenylalanine, xylose, arabinose, $\alpha$-aminoadipic acid, lysine, 1-hexadecanol, tyrosine, myo-inositol, uridine, glycine, and leucine may be detected in addition to the 14 kinds of compounds, and all of the 31 kinds of detection amounts obtained by the detection step may be used in the use step. This evaluation method enables more accurate prediction of whether or not a subject suffers from NAFLD in the future.

(Clause 5) In the evaluation method according to any one of clauses 1 to 4, in the use step, at least a value obtained by multiplying each of the detection amounts of the 14 kinds of compounds obtained in the detection step by a predetermined coefficient for each of the 14 kinds of compounds may be added. This evaluation method enables more accurate prediction of whether or not a subject will suffer from NAFLD in the future.

(Clause 6) In the evaluation method according to any one of clauses 1 to 5, in the detection step, concentrations of the 14 kinds of compounds or derivatives thereof may be obtained. This evaluation method enables more accurate prediction of whether or not a subject will suffer from NAFLD in the future.

(Clause 7) In the evaluation method according to clause 6, the derivative may be derivatized by silylation, acylation, esterification, or alkylation. According to this evaluation method, since derivatives can be detected with high sensitivity, it is possible to more accurately predict whether or not the subject suffers from NAFLD in the future.

(Clause 8) In the evaluation method according to any one of clauses 1 to 7, the detection step may be performed by gas chromatography, liquid chromatography, mass spectrometry, gas chromatography/mass spectrometry, liquid chromatography/mass spectrometry, nuclear magnetic resonance spectroscopy, or immunological detection. According to this evaluation method, since a detection method with good sensitivity is used, it is possible to more accurately predict whether or not the subject suffers from NAFLD in the future.

(Clause 9) In the evaluation method according to any one of clauses 1 to 8, the body fluid may be blood. According to this evaluation method, since the measurement sample is easily obtained and the invasiveness to the subject is low, the burden on the diagnosis of the subject can be reduced.

(Clause 10) A biomarker for evaluating an onset risk of n NAFLD according to an aspect may contain 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan. According to this biomarker, it is possible to predict whether or not the subject suffers from NAFLD in the future.

(Clause 11) The biomarker for evaluating an onset risk of NAFLD according to clause 10 may contain 15 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, and glycine. This biomarker enables more accurate prediction of whether or not a subject suffers from NAFLD in the future.

(Clause 12) The biomarker for evaluating an onset risk of NAFLD according to clause 10 may contain 21 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, 3-hydroxybutyric acid, leucine, glycine, methionine, glucose, tyrosine, and myo-inositol. This biomarker enables more accurate prediction of whether or not a subject suffers from NAFLD in the future.

(Clause 13) The biomarker for evaluating an onset risk of NAFLD according to clause 10 may contain 31 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, pyruvic acid, 2-aminobutyric acid, succinic acid, threonine, decanoic acid, methionine, phenylalanine, xylose, arabinose, $\alpha$-aminoadipic acid, lysine, 1-hexadecanol, tyrosine, myo-inositol, uridine, glycine, and leucine. This biomarker enables more accurate prediction of whether or not a subject suffers from NAFLD in the future.

<Example>

In the following example, GC/MS is performed using a serum (NAFLD preliminary group) obtained from a patient in which a healthy individual is diagnosed with NAFLD by abdominal ultrasound findings within 3 years and a serum (normal group) obtained from a patient who is a healthy individual for 3 years, and the analysis result of searching for components in serum having different concentrations in the NAFLD preliminary group and the normal group will be described.

(Subject)

Among those randomly extracted from Japanese medical examinees who visited a hospital from October 2015 to September 2016, serum of 4,495 individuals who satisfied the eligibility criteria was used as a sample. The eligibility criteria required that subjects did not meet any of the conditions listed in (1) to (5) below.

(1) Person who has expressed disagreement with participation in study
(2) Persons whose alcohol intake is 30 g/day or more in terms of ethanol for males and 20 g/day or more in terms of ethanol for females
(3) Person with present medical history during treatment of liver disease
(4) Person with present medical history during treatment for at least one of diabetes, dyslipidemia, and hyperuricemia
(5) Person with present medical history during treatment for malignant disease

All the individuals who satisfied the eligibility criteria had abdominal ultrasound findings in a complete medical checkup, and the results of analyzing clinical information and serum were cross-referenced under the consent of the Research Ethics Review Committee in the hospital.

(Pretreatment)

After blood was collected from the subject, serum was separated by centrifugation, and frozen at -80°C within 6 hours from the blood collection. The serum after cryopreservation was left at 25°C for 5 minutes to thaw. Thawed serum was subjected to centrifugation, and the supernatant was collected in a tube containing a defined amount of 40 kinds of stable isotopes (Table 1 shows only stable isotopes corresponding to 31 kinds of metabolites among stable isotopes corresponding to 40 kinds of metabolites to be measured). To the collected serum, 250 $\mu$L of a mixed solvent of chloroform (methanol/water/chloroform =2.5:1:1), and 6 $\mu$L of an internal standard solution (2-isopropylmalic acid; 0.1 mg/mL) and centrifugation was performed, and then 140 $\mu$L of water was further mixed and shaken and centrifugation was performed to obtain a sample containing an internal standard and a stable isotope. After being concentrated using a centrifugal evaporator under the condition of room temperature for 60 minutes, 180 $\mu$L of this sample was left at -80°C for 30 minutes to be frozen and was then dried overnight using a freeze dryer.

[Table 1]

| Stable Isotope |
| --- |
| Pyruvic acid (1-13C, 99%) |
| Sodium L-Lactate-2-d1 |
| DL-2-Aminobutyric-d6 Acid |
| N-Acetyl-d3-glycine-2,2-d2 |
| Succinic acid (13C4, 99%) |
| Pentanedioic-d6 Acid |
| Decanoic acid-1,2-13C2, 99 atom % 13C |
| $\alpha$-Ketoglutaric acid (13C5, 99%) |
| Creatinine-d3 (methyl-d3) |
| D-Xylose (1-13C5, 99%) |
| D-Arabinose-$^{13}$C$_5$, ≥99% $^{13}$C, ≥98% (CP) |
| DL-$\alpha$-Aminoadipic-2,5,5-d3 Acid |

(continued)

| Stable Isotope |
| --- |
| D-Fructose (13C6, 99%) |
| n-Hexadecyl-1,1,2,2-d4 Alcohol |
| Uric acid (1,3-15N2, 98%) |
| myo-Inositol-1,2,3,4,5,6-d6 |
| Uridine-5,6-d2 |
| L-Alanine (15N, 98%) |
| Glycine (15N, 98%) |
| L-Leucine (15N, 98%) |
| L-Isoleucine (15N, 98%) |
| L-Proline (15N, 98%) |
| L-Serine (15N, 98%) |
| L-Threonine (15N, 98%) |
| L-Methionine (15N, 98%) |
| L-Glutamic acid (15N, 98%) |
| L-Phenylalanine (15N, 98%) |
| L-Asparagine·H2O (15N2, 98%) |
| L-Lysine·2HCl (15N2, 98%) |
| L-Tyrosine (15N, 98%) |
| L-Tryptophan (15N2, 98%) |

(Derivatization)

The dried sample was sequentially mixed with 80 μL of a pyridine solution containing methoxyamine hydrochloride (20 mg/mL) and 40 μL of N-methyl-N-trimethylsilyltrifluoroacetamide, subjected to shaking, and then centrifuged, and 50 μL of the supernatant was transferred to an autosampler vial for GC/MS analysis.

(GC/MS Measurement)

Gas chromatography/mass spectrometry was performed on the sample under the following conditions.

<Conditions of Gas Chromatography>

System: GCMS-TQ8040 (manufactured by Shimadzu Corporation)
Column oven temperature: 80°C
Injection temperature: 280°C
Injection mode: Split injection method
Carrier gas: Helium
Carrier gas flow rate: 39 cm/sec
Separation column: DB-5 (manufactured by Agilent Technologies)
Stationary phase: (5%-phenyl)-methylpolysiloxane (nonpolar)
Column length: 30.0 m, column inner diameter: 0.25 mm, column film thickness: 1.00 μm
Column temperature program: 80°C (0 min)/80°C (2.5 min)/280°C (18.5 min)/280°C (23.0 min)

<Conditions of Mass Spectrometry >

System: GCMS-TQ8040 (manufactured by Shimadzu Corporation)
Ion source temperature: 200°C
Interface temperature: 250°C
Detector voltage: 0.2 kV
Measurement mode: Multiple Reaction Monitoring (MRM) mode
Scanning range: m/z 82-500

(Calculation of Detection Amount)

A mass spectrum at each retention time was output from the detection intensity obtained by scanning m/z at each retention time. Based on mass spectrum data in serum samples obtained in the past, metabolites (40 kinds) corresponding to each peak in the mass spectrum and stable isotopes corresponding to the metabolites were identified. The detection intensity corresponding to each metabolite was standardized using the detection intensity of the stable isotope corresponding to each metabolite, and the concentration (unit: μg/mL) in the sample was calculated as the detection amount.

(Data Analysis)

Based on the clinical diagnosis information and the detection amount obtained by GC/MS, a combination of metabolites capable of discriminating between the normal group and the onset group was examined using LASSO, which is one of machine learning techniques. Specifically, before the LASSO run, subjects were divided into two groups: a training set and a test set, and a predictive discriminant was developed in the training set to perform the validation on the test set. In the LASSO run, logistic regression with 10-fold cross-validation implemented was applied.
As a result, as listed in Table 2 below, 31 kinds of compounds were selected as distinguishable metabolites, the regression coefficients thereof were calculated, and a predictive discriminant was derived as described in the following formulae (1) and (2) to calculate the relationship between the hazard ratio and the incidence as listed in Table 3. Note that, for the predictive discriminant, a formula obtained by adding a predetermined constant (first term on the right side) and the sum of the respective compound detection amounts multiplied by the respective coefficients (second term on the right side) is the prediction score (the following formula (1)). This prediction score is a logarithmic hazard ratio, and by calculating 1e [prediction score], the hazard ratio (hazard ratio relative to the median of hazard in the general population with the onset of NAFLD) is derived. This hazard ratio is a hazard ratio for each individual when the hazard of the general population is reference (hazard ratio = 1), and indicates that the higher the value, the higher the onset risk of NAFLD. Specific criteria are shown in Table 3.

[Table 2]

| Metabolite | Coefficient | Metabolite | Coefficient |
|---|---|---|---|
| Constant K | -2.40391 | Glutamic acid-3TMS | 2.75080 |
| Pyruvic acid-methyloxime-TMS | -3.62478 | Phenylalanine-2TMS | 0.31922 |
| Lactic acid-2TMS | 0.49902 | Xylose-methyloxime-4TMS | -14.35933 |
| Alanine-2TMS | 1.29249 | Arabinose-methyloxime-4TMS | -0.01182 |
| 2-Aminobutyric acid-2TMS | -0.33578 | Asparagine-3TMS | -0.41965 |
| Acetylglycine-TMS | -0.01481 | $\alpha$-Aminoadipic acid-3TMS | -0.01972 |
| Isoleucine-2TMS | 0.75419 | Fructose-methyloxime-5TMS | -0.03558 |
| Succinic acid-2TMS | 0.04018 | Lysine-4TMS | -0.08390 |
| Proline-2TMS | 0.03107 | 1-Hexadecanol-TMS | 1.51069 |
| Serine-3TMS | -0.80263 | Tyrosine-3TMS | 0.37611 |
| Threonine-3TMS | 0.96646 | Uric acid-4TMS | 0.25277 |
| Glutaric acid-2TMS | -0.03950 | myo-Inositol-6TMS | -2.25953 |
| Decanoic acid-TMS | -0.77793 | Uridine-4TMS | -0.12601 |

(continued)

| Metabolite | Coefficient | Metabolite | Coefficient |
|---|---|---|---|
| Methionine-2TMS | -0.35423 | Glycine-2TMS | -0.19429 |
| $\alpha$-Ketoglutaric acid -methyloxime-2TMS | -0.18526 | Leucine-2TMS | -0.00320 |
| Creatinine-3TMS | 0.39279 | Tryptophan-2TMS | -0.20818 |

[Equation 2]

$$[\text{Prediction Score}] = [\text{Constant K}] + \sum([\text{Coefficient of each compound}] \times [\text{Detection amount of each compound}]) \cdots (1)$$

$$[\text{Hazard ratio}] = e^{[\text{Prediction Score}]} \cdots (2)$$

TMS in Table 2 denotes the trimethylsilylated derivative. In Formula 1, the second term on the right side is the sum of 31 kinds of compounds, the constant K and the coefficient of each compound are as shown in Table 2, and the detection amount of each compound is the concentration µg of the specific compound present in 1 mL of serum.

[Table 3]

| Hazard ratio | Incidence/year | 95% Confidence interval | | 2-year incidence | 3-year incidence |
|---|---|---|---|---|---|
| | | Lower limit | Upper limit | | |
| 0.5 or less | 0.4% | 0.1% | 1.8% | 0.8% | 1.2% |
| 0.5 or more and 1 or less | 1.1% | 0.7% | 1.8% | 2.2% | 3.2% |
| 1 or more and 2 or less | 2.9% | 2.1% | 4.2% | 5.8% | 8.5% |
| 2 or more and 4 or less | 7.6% | 5.3% | 10.8% | 14.6% | 21.1% |
| more than 4 | 10.0% | 5.9% | 16.8% | 19.0% | 27.1% |

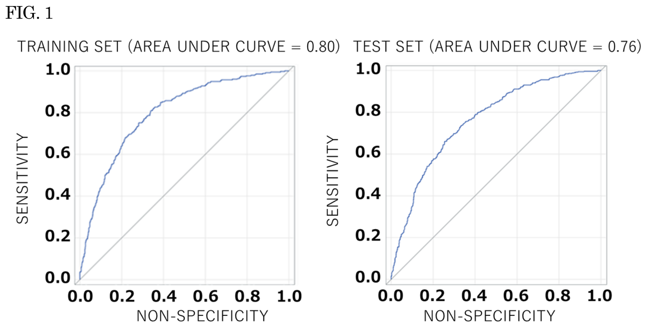

Time-dependent receiver operating characteristic (ROC) analysis was performed on the predictive discriminant and the results are shown in FIG. 1. For quantitative evaluation, when the area under the curve (AUC) at this time was calculated, good values of 0.80 and 0.76 were obtained in the training set and the test set, respectively. Therefore, it can be seen that the evaluation method of the embodiment has good identification performance. In addition, since it is based on the data of 4,495 people including a large number of normal groups and NAFLD preliminary groups, the reliability is high.

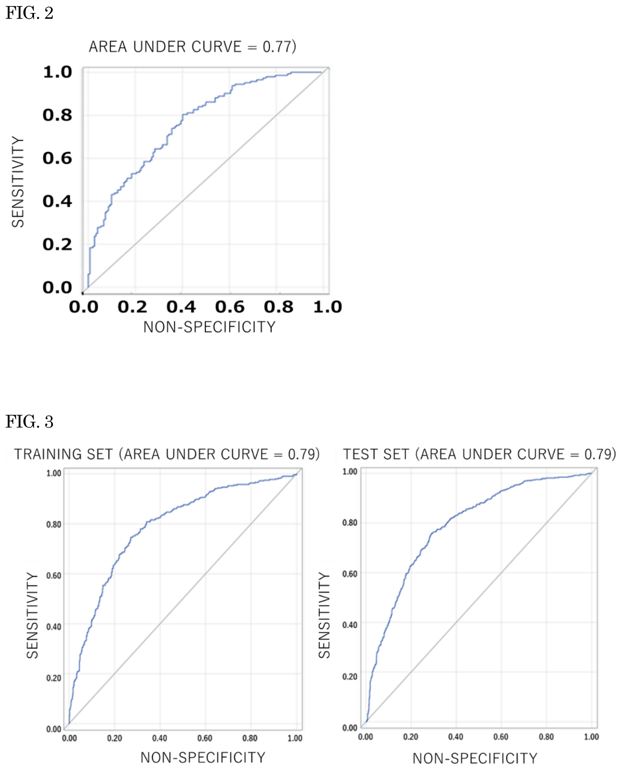

In addition, as another cohort, among persons randomly extracted from Japanese medical examinees who visited a hospital at another time, serum of 862 persons who satisfied the above eligibility criteria was used as a sample. ROC analysis was also performed for this additional cohort and the results are shown in FIG. 2. The AUC at this time was 0.77.

<Example 2>

Values of 34 kinds of metabolites (specific compounds) were subjected to the LASSO method in the same manner as in Example 1 (cohort of 4,495 people), except that 6 kinds of metabolites (Leucine-TMS, Decanoic acid-TMS, 1-Hexadecanol-TMS, Uridine-4TMS, 2-Aminoadipic acid-3TMS, and Histidine-3TMS) with unstable elements were excluded from the above 40 kinds of metabolites to enhance the stability of the analysis. As a result, 21 kinds of metabolites were selected as distinguishable metabolites as shown in Table 4 below, and their regression coefficients were calculated, and the relationship between the hazard ratio and the incidence rate was calculated as shown in Table 5 below.

[Table 4]

| Compound | Coefficient | Compound | Coefficient |
|---|---|---|---|
| Constant K | -0.87955 | Methionine-2TMS | -0.02911 |
| Lactic acid-2TMS | 0.42707 | $\alpha$-Ketoglutaric acid-methyloxime-2TMS | 0.84303 |

(continued)

| Compound | Coefficient | Compound | Coefficient |
|---|---|---|---|
| Alanine-2TMS | 1.14453 | Creatinine-3TMS | 0.96745 |
| 3-Hydroxybutyric acid-2TMS | -0.02811 | Glutamic acid-3TMS | 2.42947 |
| Leucine-2TMS | 0.00606 | Asparagine-3TMS | -0.63128 |
| Acetylglycine-TMS | -1.42833 | Fructose-methyloxime-5TMS | -26.44789 |
| Isoleucine-2TMS | 0.27348 | Glucose-methyloxime-5TMS | 1.76058 |
| Proline-2TMS | 0.11992 | Tyrosine-3TMS | 0.01743 |
| Glycine-3TMS | -0.50910 | Uric acid-4TMS | 0.26972 |
| Serine-3TMS | -0.29387 | myo-Inositol-6TMS | -0.41412 |
| Glutaric acid-2TMS | 0.15692 | Tryptophan-2TMS | -0.77229 |

[Table 5]

| Hazard ratio | Incidence/year | 95% Confidence interval | | 2-year incidence | 3-year incidence |
|---|---|---|---|---|---|
| | | Lower limit | Upper limit | | |
| 0.5 or less | 0.3% | 0.1% | 0.7% | 0.5% | 0.8% |
| 0.5 or more and 1 or less | 1.2% | 0.5% | 1.8% | 2.3% | 3.5% |
| 1 or more and 2 or less | 3.1% | 2.3% | 4.3% | 6.2% | 9.1% |
| 2 or more and 4 or less | 6.8% | 4.9% | 9.5% | 13.1% | 19.0% |
| more than 4 | 19.1% | 13.8% | 26.4% | 34.5% | 47.0% |

ROC analysis was performed using the 21 kinds of metabolites in the same manner as in Example 1. The AUC was 0.79 for both the training and test sets, indicating strong predictive performance. Results are shown in FIG. 3.

<Example 3>

Values of 15 kinds of metabolites (specific compounds) were subjected to the LASSO method in the same manner as in Example 1 (cohort of 4,495 people), except that 6 kinds of metabolites (Leucine-TMS, Decanoic acid-TMS, 1-Hexadecanol-TMS, Uridine-4TMS, 2-Aminoadipic acid-3TMS, and Histidine-3TMS) with unstable elements were excluded from the above 40 kinds of metabolites, and furthermore, a mode set to reduce the number of metabolites was run. As a result, 15 kinds of metabolites were selected as distinguishable metabolites as shown in Table 6, and their regression coefficients were calculated, and the relationship between the hazard ratio and the incidence rate was calculated as shown in Table 7.

[Table 6]

| Compound | Coefficient | Compound | Coefficient |
|---|---|---|---|
| Constant K | -0.93492 | Glutaric acid-2TMS | 0.10929 |
| Lactic acid-2TMS | 0.41309 | $\alpha$-Ketoglutaric acid-methyloxime-2TMS | 0.27619 |
| Alanine-2TMS | 1.16622 | Creatinine-3TMS | 0.31658 |
| Acetylglycine-TMS | -1.16451 | Glutamic acid-3TMS | 2.83357 |
| Isoleucine-2TMS | 0.01125 | Asparagine-3TMS | -0.24578 |
| Proline-2TMS | 0.15301 | Fructose-methyloxime-5TMS | -11.92795 |
| Glycine-3TMS | -0.52138 | Uric acid-4TMS | 0.24772 |
| Serine-3TMS | -0.23099 | Tryptophan-2TMS | -0.57015 |

[Table 7]

| Hazard ratio | Incidence/year | 95% Confidence interval | | 2-year incidence | 3-year incidence |
|---|---|---|---|---|---|
| | | Lower limit | Upper limit | | |
| 0.5 or less | 0.3% | 0.1% | 0.9% | 0.6% | 1.0% |
| 0.5 or more and 1 or less | 1.0% | 0.7% | 1.6% | 2.1% | 3.1% |
| 1 or more and 2 or less | 3.9% | 2.9% | 5.1% | 7.6% | 11.2% |
| 2 or more and 4 or less | 7.7% | 5.6% | 10.5% | 14.8% | 21.4% |
| more than 4 | 15.2% | 9.9% | 23.4% | 28.1% | 39.0% |

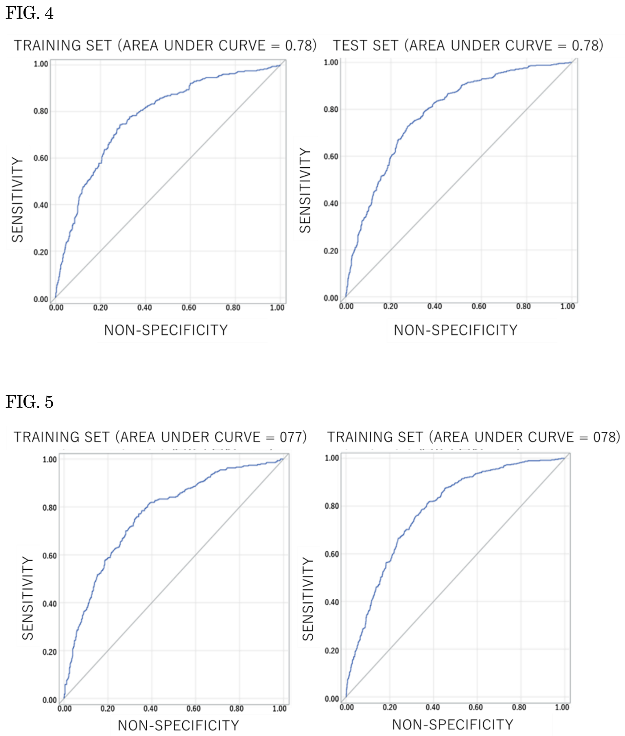

ROC analysis was performed using the 15 kinds of metabolites in the same manner as in Example 1. The AUC was 0.78 for both the training and test sets, demonstrating reliable predictive capability. Results are shown in FIG. 4.

<Example 4>

The 14 kinds of metabolites common to Examples 1 through 3 were selected as distinguishable metabolites (see Table 8). In addition, as shown in Table 9, the relationship between the hazard ratio and the incidence rate was calculated.

[Table 8]

| Compound | Coefficient | Compound | Coefficient |
|---|---|---|---|
| Constant K | 2.16984 | | |
| Lactic acid-2TMS | 0.41309 | $\alpha$-Ketoglutaric acid-methyloxime-2TMS | 0.27619 |
| Alanine-2TMS | 1.16622 | Creatinine-3TMS | 0.31658 |
| Acetylglycine-TMS | -1.16451 | Glutamic acid-3TMS | 2.83357 |
| Isoleucine-2TMS | 0.01125 | Asparagine-3TMS | -0.24578 |
| Proline-2TMS | 0.15301 | Fructose-methyloxime-5TMS | -11.92795 |
| Serine-3TMS | -0.23099 | Uric acid-4TMS | 0.24772 |
| Glutaric acid-2TMS | 0.10929 | Tryptophan-2TMS | -0.57015 |

[Table 9]

| Hazard ratio | Incidence/year | 95% Confidence interval | | 2-year incidence | 3-year incidence |
|---|---|---|---|---|---|
| | | Lower limit | Upper limit | | |
| 0.5 or less | 0.2% | 0.1% | 0.8% | 0.5% | 0.7% |
| 0.5 or more and 1 or less | 1.1% | 0.7% | 1.7% | 2.2% | 3.3% |
| 1 or more and 2 or less | 4.1% | 3.1% | 5.3% | 8.0% | 11.7% |
| 2 or more and 4 or less | 7.3% | 5.3% | 10.1% | 14.1% | 20.4% |
| more than 4 | 14.2% | 9.0% | 22.5% | 26.4% | 36.9% |

ROC analysis was performed in the same manner as in Example 1 using the 14 kinds of specific compounds. The AUC was 0.77 for the training set and 0.78 for the test set, indicating consistent and robust predictive performance. Results are shown in FIG. 5.

Claims

1.    A method for evaluating an onset risk of nonalcoholic fatty liver disease, the method comprising:

an acquisition step of acquiring a sample derived from a body fluid of a subject;

a detection step of detecting 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan in the sample; and

a use step of using all detection amounts of the 14 kinds of compounds obtained in the detection step.

2. The method for evaluating an onset risk according to claim 1, the method comprising:

detecting, in addition to the 14 kinds of compounds, glycine in the detection step; and
using, in the use step, all detection amounts of 15 kinds of compounds obtained in the detection step.

3. The method for evaluating an onset risk according to claim 1, the method comprising:

detecting, in addition to the 14 kinds of compounds, 3-hydroxybutyric acid, leucine, glycine, methionine, glucose, tyrosine, and myo-inositol in the detection step; and
using, in the use step, all detection amounts of 21 kinds of compounds obtained in the detection step.

4. The method for evaluating an onset risk according to claim 1, the method comprising:

detecting, in addition to the 14 kinds of compounds, pyruvic acid, 2-aminobutyric acid, succinic acid, threonine, decanoic acid, methionine, phenylalanine, xylose, arabinose, $\alpha$-aminoadipic acid, lysine, 1-hexadecanol, tyrosine, myo-inositol, uridine, glycine, and leucine in the detection step; and
using, in the use step, all detection amounts of 31 kinds of compounds obtained in the detection step.

5. The method for evaluating an onset risk according to claim 1, the method comprising, in the use step, at least summing values obtained by multiplying each of the detection amounts of the 14 kinds of compounds by a predetermined coefficient for each of the 14 kinds of compounds.

6. The method for evaluating an onset risk according to claim 1, the method comprising, in the detection step, obtaining concentration of each of the 14 kinds of compounds or a derivative thereof.

7. The method for evaluating an onset risk according to claim 6, wherein the derivative is derivatized by silylation, acylation, esterification, or alkylation.

8. The method for evaluating an onset risk according to claim 1, wherein the detection step is performed by gas chromatography, liquid chromatography, mass spectrometry, gas chromatography/mass spectrometry, liquid chromatography/mass spectrometry, nuclear magnetic resonance spectroscopy, or immunological detection.

9. The method for evaluating an onset risk according to claim 1, wherein the body fluid is blood.

10. A biomarker for evaluating an onset risk of nonalcoholic fatty liver disease, the biomarker comprising 14 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, and tryptophan.

11. The biomarker according to claim 10, comprising 15 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, and glycine.

12. The biomarker according to claim 10, comprising 21 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, 3-hydroxybutyric acid, leucine, glycine, methionine, glucose, tyrosine, and myo-inositol.

13. The biomarker according to claim 10, comprising 31 kinds of compounds consisting of lactic acid, alanine, acetylglycine, isoleucine, proline, serine, glutaric acid, $\alpha$-ketoglutaric acid, creatinine, glutamic acid, asparagine, fructose, uric acid, tryptophan, pyruvic acid, 2-aminobutyric acid, succinic acid, threonine, decanoic acid, methionine, phenylalanine, xylose, arabinose, $\alpha$-aminoadipic acid, lysine, 1-hexadecanol, tyrosine, myo-inositol, uridine, glycine, and leucine.

FIG. 1

TRAINING SET (AREA UNDER CURVE = 0.80)    TEST SET (AREA UNDER CURVE = 0.76)

FIG. 2

AREA UNDER CURVE = 0.77)

FIG. 3

TRAINING SET (AREA UNDER CURVE = 0.79)

TEST SET (AREA UNDER CURVE = 0.79)

FIG. 4

FIG. 5

## INTERNATIONAL SEARCH REPORT

| International application No. |
| --- |
| **PCT/JP2024/017846** |

| A. | CLASSIFICATION OF SUBJECT MATTER |
| --- | --- |

*G01N 33/50*(2006.01)i
FI: G01N33/50 D

According to International Patent Classification (IPC) or to both national classification and IPC

| B. | FIELDS SEARCHED |
| --- | --- |

Minimum documentation searched (classification system followed by classification symbols)

G01N33/50

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | WO 2020/066162 A1 (SHIMADZU CORPORATION) 02 April 2020 (2020-04-02) paragraphs [0020]-[0027], [0034]-[0056] | 1-13 |
| Y | WO 2013/002381 A1 (AJINOMOTO CO., INC.) 03 January 2013 (2013-01-03) paragraphs [0017], [0021], [0027], [0080], [0084] | 1-13 |
| Y | JP 2016-065873 A (METANOMICS HEALTH GMBH) 28 April 2016 (2016-04-28) pp. 26-38, tables 1A-5A | 1-13 |
| Y | JP 2018-502286 A (METABOLON, INC.) 25 January 2018 (2018-01-25) paragraphs [0119]-[0120], [0144], [0151], [0160], [0171], [0177], [0190]-[0192], tables 2, 3, 13, 18, 24, 30, 41 | 1-13 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **13 June 2024** | **25 June 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
**Information on patent family members**

International application No.

**PCT/JP2024/017846**

| Patent document cited in search report | | | Publication date (day/month/year) | Patent family member(s) | Publication date (day/month/year) |
|---|---|---|---|---|---|
| WO | 2020/066162 | A1 | 02 April 2020 | (Family: none) | |
| WO | 2013/002381 | A1 | 03 January 2013 | US 2014/0113378 A1 in particular, abstract, claims | |
| JP | 2016-065873 | A | 28 April 2016 | US 2013/0126722 A1 in particular, paragraphs [0011]-[0015], tables 1A-5A EP 2863227 A1 | |
| JP | 2018-502286 | A | 25 January 2018 | US 2017/0370954 A1 in particular, tables 2, 3, 13, 18, 24, 30, 41 CN 107002113 A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020034561 A
- JP 2020528138 W
- JP 2012528340 W
- JP 2020120625 A